# EUROPEAN PATENT APPLICATION

(11) **EP 2 724 665 A2**
(43) Date of publication of application: **30.04.2014**
(21) Application number: 13184315.3
(22) Date of filing: 13.09.2013
(51) Int. Cl.: A61B 5/00, G01N 29/24

(54) **Object information acquiring apparatus and cover for photoacoustic probe**

(30) Priority: 23.10.2012 JP 2012233900
(71) Applicant: Canon Kabushiki Kaisha, Tokyo 146-8501 (JP)
(72) Inventor: Tokita, Toshinobu, Tokyo, Tokyo 146-8501 (JP); Yamaguchi, Sakiko, Tokyo, Tokyo 146-8501 (JP)
(74) Representative: TBK

(57) **Abstract**

There is used an object information acquiring apparatus that has a light source; a photoacoustic probe including an emission end that emits light from the light source to an object and a reception section that receives an acoustic wave generated from the object to which the light is emitted; a cover covering at least the emission end of the photoacoustic probe; a light-shielding member provided inside the cover and filling a gap formed between the photoacoustic probe and the cover when the photoacoustic probe is inserted into the cover; and a processor acquiring information on the inside of the object based on the acoustic wave.

## Description

### BACKGROUND OF THE INVENTION

### Field of the Invention

The present invention relates to an object information acquiring apparatus and a cover for a photoacoustic probe.

### Description of the Related Art

As a method for specifically imaging vascularization resulting from a cancer, attention is paid to photoacoustic tomography (hereinafter referred to as PAT). The PAT is a method in which illumination light (near infrared rays) is emitted to an object, a photoacoustic wave emitted from the inside of the object is received by an ultrasonic probe, and the received photoacoustic wave is imaged.

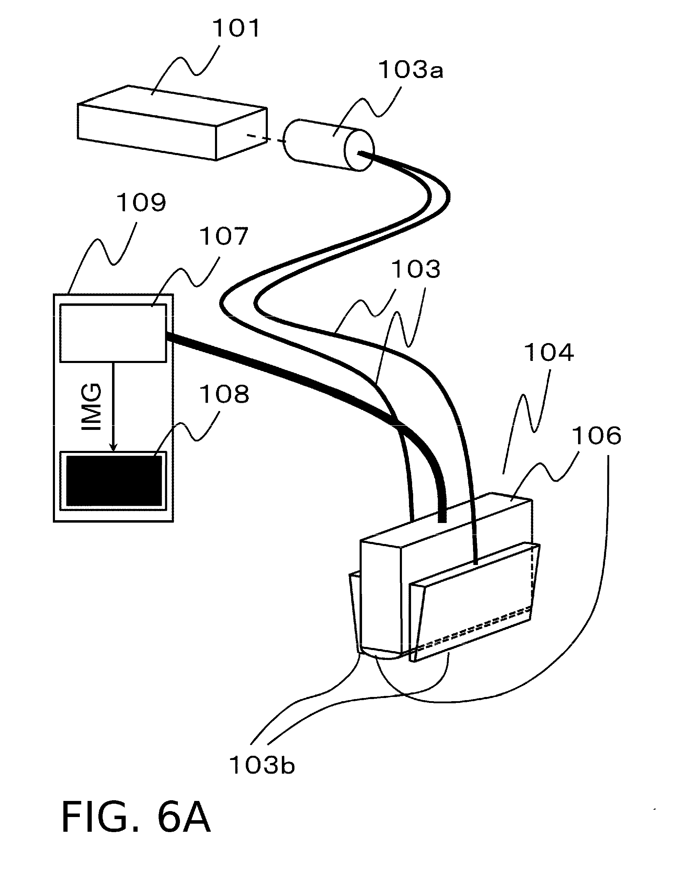

FIG. 6A is a schematic diagram of a handheld photoacoustic apparatus described in technique by S.A. Ermilov et al., in "Development of laser optoacoustic and ultrasonic imaging system for breast cancer utilizing handheld array probes", Photons Plus Ultrasound: Imaging and Sensing 2009, Proc. of SPIE vol. 7177, 2009. A photoacoustic probe 104 has a structure in which a reception section 106 for receiving the photoacoustic wave is sandwiched by illumination optical systems including emission ends 103b of bundle fibers 103. Illumination light from a light source 101 enters into the bundle fibers 103 from an incident end 103a, and is emitted to the object from the emission ends 103b. Subsequently, the photoacoustic wave generated from the object by a photoacoustic effect is received by the reception section 106, and is converted to an electrical signal. A processor 107 of an ultrasonic device 109 performs amplification, digitization, and image reconstruction on the electrical signal to thereby generate image information (IMG), and transmits the image information to a display device 108. With this, a photoacoustic image indicative of characteristic information on the inside of the object is displayed.

In the abovementioned "Development of laser optoacoustic and ultrasonic imaging system for breast cancer utilizing handheld array probes", in a case where the illumination light is emitted when the photoacoustic probe is not used (e.g., when the photoacoustic probe is not in contact with the object), the illumination light having a relatively high energy density is released into the air. Consequently, when the photoacoustic probe is not used, it is preferable to cover at least the emission end of the photoacoustic probe.

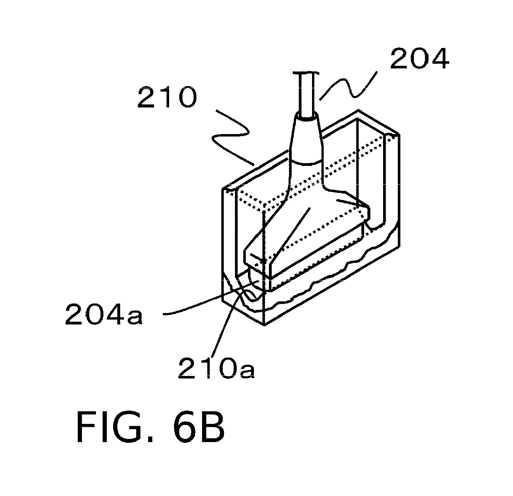

Next, in Japanese Patent Application Laid-open No. H7-327996 shown in FIG. 6B, an ultrasonic probe can be accommodated. In FIG. 6B, a probe 204 is accommodated in a holder 210. A space is formed such that a holder bottom surface 210a does not interfere with an acoustic lens 204a at the tip of the probe 204 when the probe 204 is accommodated in the holder 210.

Patent Literature 1: Japanese Patent Application Laid-open No. H7-327996

Non Patent Literature 1: S.A. Ermilov et al., in "Development of laser optoacoustic and ultrasonic imaging system for breast cancer utilizing handheld array probes", Photons Plus Ultrasound: Imaging and Sensing 2009, Proc. of SPIE vol. 7177, 2009

### SUMMARY OF THE INVENTION

However, the conventional art has had the following problems.
The use of the folder (cover) in Japanese Patent Application Laid-open No. H7-327996 can cope with the issue of the light irradiation into the air when the probe is not used. However, in Japanese Patent Application Laid-open No. H7-327996 there is no description of a gap formed between the inner wall of the holder 210 and the probe 204 when the probe 204 is accommodated in the holder 210. Consequently, in a case where the holder 210 of Japanese Patent Application Laid-open No. H7-327996 is applied to the abovementioned "Development of laser optoacoustic and ultrasonic imaging system for breast cancer utilizing handheld array probes", and the photoacoustic probe 104 is accommodated in the holder, when the illumination light is emitted while the photoacoustic probe 104 is accommodated, the light is reflected and scattered in the holder 210, and is released into the air.

In addition, in Japanese Patent Application Laid-open No. H7-327996, the holder 210 is formed of a relatively elastic material such as silicone rubber or urethane foam. However, in this case, when the photoacoustic probe 104 is accommodated in the holder 210, the holder 210 is deformed by the weight of the photoacoustic probe 104, and the gap between the photoacoustic probe 104 and the holder 210 is increased in size. As a result, the illumination light becomes more likely to be released into the air. Consequently, even when the technique of Japanese Patent Application Laid-open No. H7-327996 is applied to the abovementioned "Development of laser optoacoustic and ultrasonic imaging system for breast cancer utilizing handheld array probes", there has been room for improvement.

The present invention has been achieved in view of the above problems. This invention is developed to prevent the light irradiation from the gap between the photoacoustic probe and the cover.

The present invention in its first aspect provides an object information acquiring apparatus as specified in claim 1 and 2.

The present invention in its second aspect provides a cover for photoacoustic probe as specified in claim 3.

The present invention in its third aspect provides an object information acquiring apparatus as specified in claims 4 to 7.

The present invention in its fourth aspect provides an object information acquiring apparatus as specified in claims 8 to 16.

According to the present invention, it is possible to prevent the light irradiation from the gap between the photoacoustic probe and the cover.
Further features of the present invention will become apparent from the following description of exemplary embodiments with reference to the attached drawings.

### BRIEF DESCRIPTION OF THE DRAWINGS

FIG. 1 is a view for explaining the configuration of a photoacoustic apparatus in an embodiment of the present invention;
FIGS. 2A to 2C are views for explaining a cover of a photoacoustic probe in the embodiment of the present invention;
FIG. 3 is a view for explaining the cover of the photoacoustic probe in a first embodiment;
FIGS. 4A and 4B are views for explaining the cover of the photoacoustic probe in a second embodiment;
FIG. 5A is a view for explaining the photoacoustic probe in a third embodiment;
FIG. 5B is a flowchart for explaining a fault diagnosis process in the third embodiment;
FIG. 6A is a view for explaining the configuration of the photoacoustic device of the background art; and
FIG. 6B is a view for explaining the configuration of each of a probe and a holder of the background art.

### DESCRIPTION OF THE EMBODIMENTS

Hereinbelow, preferred embodiments of the present invention will be described with reference to the drawings. However, the dimension, material, shape, and relative arrangement of each component described below should be appropriately changed according to the configuration and various conditions of an apparatus to which the invention is applied, and are not intended to limit the scope of the invention to the following description.

An object information acquiring apparatus of the present invention includes an apparatus that utilizes a photoacoustic effect of receiving an acoustic wave generated inside an object by emitting light (an electromagnetic wave) to the object and acquiring object information as image data. The object information includes a source distribution of the acoustic wave generated by light irradiation, an initial sound pressure distribution inside the object, a light energy absorption density distribution or absorption coefficient distribution derived from the initial sound pressure distribution, and a concentration distribution of a substance constituting a tissue. Examples of the concentration distribution of the substance include an oxygen saturation distribution, an oxidized/reduced hemoglobin concentration distribution, and the like.
In the following description, a photoacoustic apparatus will be described as an example of the object information acquiring apparatus.

The acoustic wave mentioned in the present invention is typically an ultrasonic wave, and includes an elastic wave called a sound wave, the ultrasonic wave, or the acoustic wave. The acoustic wave generated by the photoacoustic effect is referred to as a photoacoustic wave or a photo-ultrasonic wave.

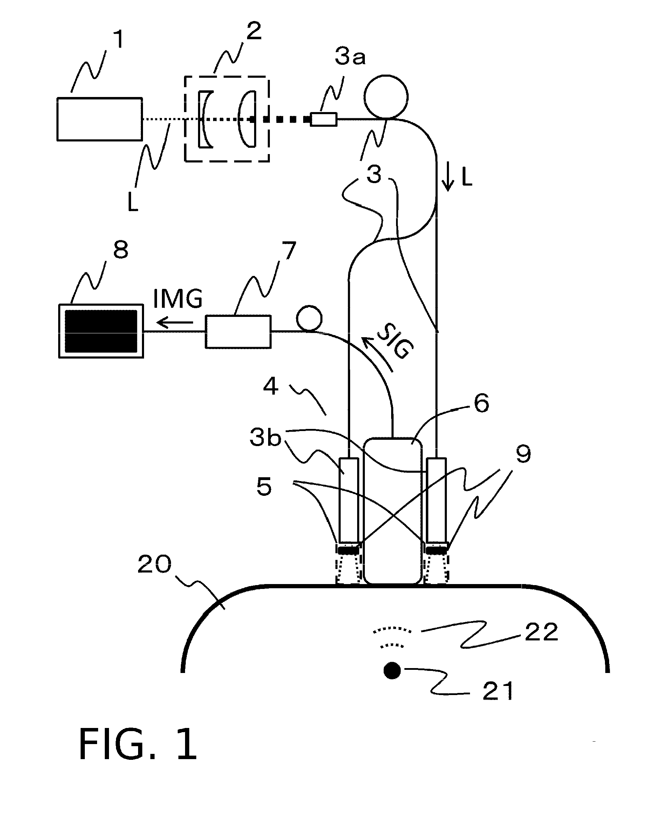

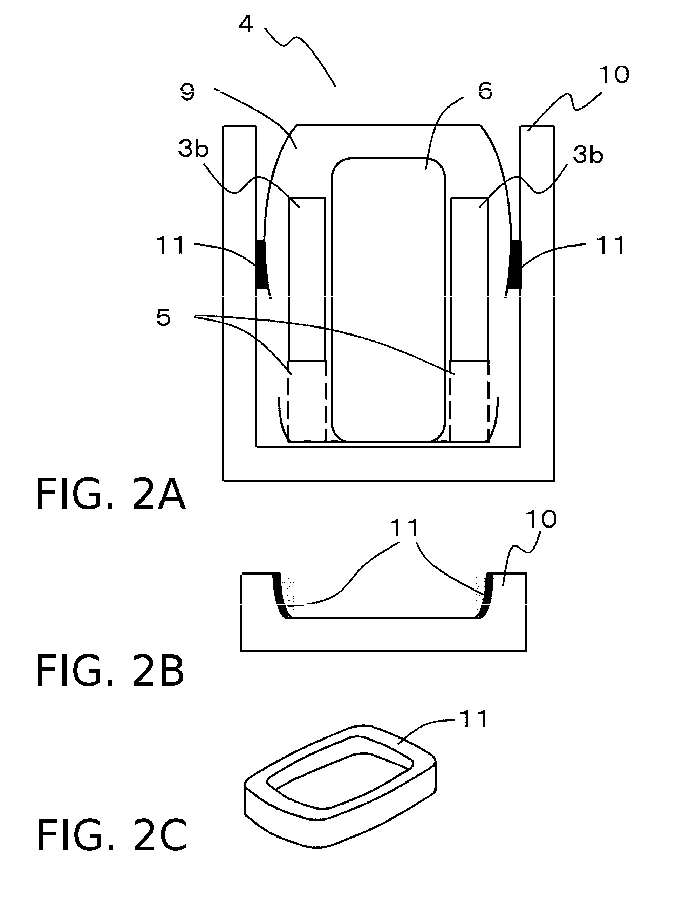

An embodiment of the present invention will be described by using FIGS. 1 and 2. FIG. 1 schematically shows the photoacoustic apparatus. In the photoacoustic apparatus, illumination light L emitted from a light source 1 is shaped by a first illumination optical system 2, and enters into an incident end 3a of a bundle fiber 3. The illumination light L is transmitted to a photoacoustic probe 4 by the bundle fiber 3, and is emitted from emission ends 3b of the bundle fiber 3.

The photoacoustic probe 4 includes the emission ends 3b, second illumination optical systems 5 that shape the illumination light emitted from the emission ends 3b, and a reception section 6 that receives the photoacoustic wave. When the illumination light having reached an object 20 via the second illumination optical systems 5 is diffused and propagated inside the object, and is absorbed by an absorbent body 21, a photoacoustic wave 22 is generated.

The reception section 6 includes an element for converting the acoustic wave to an electrical signal such as a piezoelectric element or a CMUT. Accordingly, when the reception section 6 receives the photoacoustic wave 22 propagated in the object 20, the photoacoustic wave 22 is converted to an electrical signal (SIG) by the element. Thereafter, the electrical signal (SIG) having been sent to a processor 7 is subjected to amplification, digital conversion, and filtering, and then subjected to image reconstruction by a known method, whereby image information (IMG) is generated. The image information (IMG) is sent to a display device 8, and the information on the inside of the object is displayed.

Note that, in FIG. 1, although the bundle fiber 3 branches at some midpoint to provide the emission end 3b and the second illumination optical system 5 at two locations, the number of emission ends 3b or second illumination optical systems 5 is not limited thereto. For example, it is also effective to provide the emission end 3b and the second illumination optical system 5 adjacent to only one surface of the reception section 6 without the branching, or the number thereof may be three or more.
In addition, although not shown in the drawing, covering the photoacoustic probe 4 with a housing has preferable effects such as facilitating usage and enhancing stability.

The light source 1 emits a near infrared ray preferably having a wavelength of about 600 nm to about 1100 nm. As the light source 1, it is possible to use, e.g., pulse lasers such as a Nd:YAG laser and an alexandrite laser, a Ti:sa laser using Nd:YAG laser light as excitation light, an OPO laser, and a semiconductor laser.

In FIG. 1, although the illumination light emitted by the light source 1 is transmitted to the emission ends 3b via the first illumination optical system 2 and the bundle fiber 3, the light transmission method is not limited thereto. As the light transmission method, it is also effective to combine mirrors or prisms and use the reflection or refraction thereof. Further, it is also effective to use the semiconductor laser in the light source 1 and replace the emission end 3b with the light source 1.

Note that the irradiation of the illumination light and the reception of the photoacoustic wave by the reception section 6 need to be synchronized. Accordingly, a part of the optical path between the light source 1 and the second illumination optical system 5 may be caused to branch, light may be detected by a sensor such as a photodiode or the like, and the reception section 6 may be caused to start the reception by using its detection signal as a trigger. In addition, the light emission timing of the light source 1 and the reception timing of the processor 7 may be synchronized by using a pulse generator that is not shown.

FIG. 2A is a cross-sectional view showing the photoacoustic probe 4 and a cover 10. The cover 10 in FIG. 2A is formed so as to cover the entire photoacoustic probe 4. As shown in the drawing, the entire second illumination optical systems 5 are positioned in the cover 10, and a space closer to the entrance of the cover 10 than the second illumination optical system 5 is filled with a light-shielding member 11. As a result, even when the light is emitted in a state in which the photoacoustic probe 4 is accommodated, the light does not leak to the outside of the cover 10. Further, the entire photoacoustic probe 4 is fixed, a gap becomes less likely to be formed by external forces.

On the other hand, the cover 10 in FIG. 2B is formed so as to cover at least the tip of the photoacoustic probe 4, i.e., the emission surface of the illumination light. Even with such a shape, it is possible to obtain the sufficient effect of preventing the light irradiation to the outside with the presence of the light-shielding member 11.

Note that, in each of FIGS. 2A and 2B, although the end of the photoacoustic probe 4 is oriented downward and the tip thereof is covered with the cover 10, the orientation is not limited to the downward orientation, and is arbitrarily determined.

It is preferable to use, for the cover 10, a material having relatively high rigidity such as a metal, a resin such as a plastic, or ceramic, so that no great deformation is induced when the insertion of the photoacoustic probe 4 is implemented.

The light-shielding member 11 is provided at least in a part of the inner wall of the cover 10. As shown in the perspective view of FIG. 2C, the light-shielding member 11 is preferably formed into a shape that surrounds the photoacoustic probe 4. The light-shielding member 11 is preferably formed of a deformable body as a deformable member such as rubber or hard sponge. By forming the light-shielding member 11 by using the deformable body, the gap between a part of a housing 9 as the outer periphery of the photoacoustic probe 4 and the inner wall of the cover 10 is filled by the deformation, and the light leaking to the outside is thereby reduced.

According to the configuration described above, it becomes possible to prevent the deformation of the cover 10 for the photoacoustic probe and reduce the gap between the cover 10 and the photoacoustic probe 4 by the deformation of the light-shielding member 11. As a result, by covering the tip of the photoacoustic probe 4 with the cover 10 when the measurement is not performed, it is possible to significantly reduce the light leaking to the outside even when the illumination light is emitted.

That is, the tip of the photoacoustic probe including the emission end of the illumination light is covered with the cover, and the gap between the inner wall of the cover and the photoacoustic probe is filled by the deformation of the light-shielding member. As a result, only by covering the photoacoustic probe with the cover, even when the illumination light is emitted, it becomes possible to significantly reduce the light leaking to the outside, and improve safety regarding the illumination light.

### <Embodiment 1>

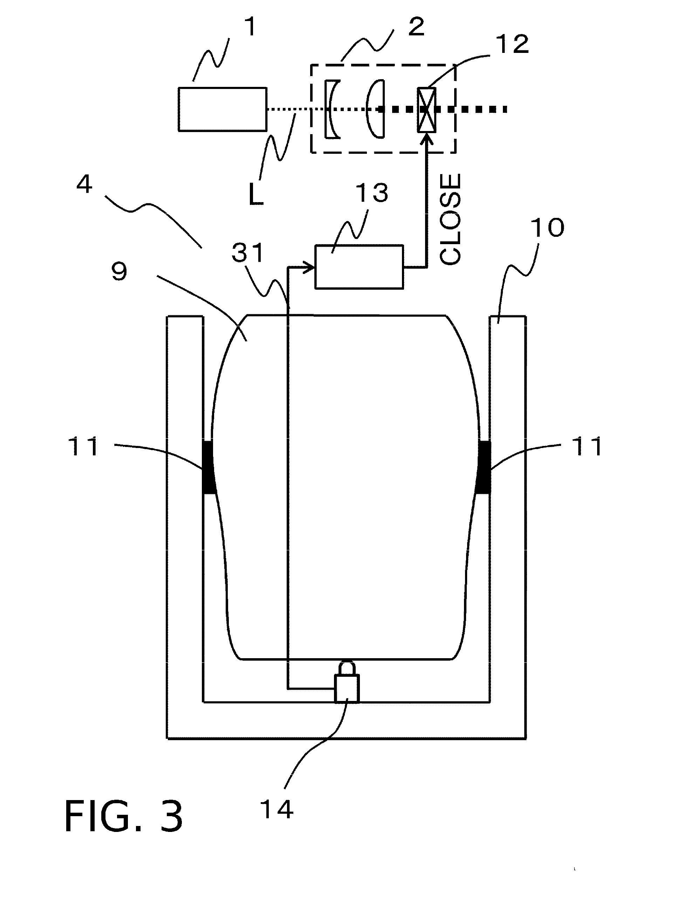

In the present embodiment, an example of the control of the light irradiation of the photoacoustic probe 4 will be described with reference to FIG. 3.

The light source 1 and the illumination light L in FIG. 3 are the same as those in FIG. 1. The first illumination optical system 2 has a shutter 12, and blocks the light by closing the shutter 12 in response to a shutter close instruction (CLOSE) from a controller 13 as described later. The photoacoustic probe 4 in FIG. 3 is considered to be substantially the same as that of FIG. 2 though the details thereof are omitted, and has a probe detection sensor 14 inside the cover 10.

The probe detection sensor 14 inside the cover 10 is provided in order to detect whether or not the tip of the photoacoustic probe 4 including the emission end of the illumination light is covered with the cover 10. The probe detection sensor 14 may be a mechanical sensor such as a limit switch, or an optical sensor or an electrostatic sensor can be used as the probe detection sensor 14. The probe detection sensor 14 sends probe cover information 31 indicative of whether or not the tip of the photoacoustic probe 4 including the emission end of the illumination light is covered with the cover 10 to the controller 13.

The controller 13 controls the opening/closing operations of the shutter 12 according to the probe cover information. That is, in a case where the probe cover information indicates that the tip of the photoacoustic probe 4 including the emission end of the illumination light is covered with the cover 10, the controller 13 closes the shutter 12. With this operation, even when the illumination light is emitted from the light source 1, the illumination light is not emitted from the emission end of the illumination light of the photoacoustic probe 4. Accordingly, it is possible to prevent the light irradiation from the emission end when the tip of the photoacoustic probe 4 including the emission end of the illumination light is covered with the cover 10, and hence the safety is improved.

In FIG. 3, the light-shielding member 11 is provided inside the cover 10. However, according to the method of the present embodiment, the light is not emitted when the photoacoustic probe 4 is accommodated, and hence it is possible to improve the safety even without the light-shielding member 11.

It goes without saying that, even in a case where the cover 10 includes the light-shielding member 11, it is possible to enhance the effect by the combination of light shielding by the light-shielding member and the suspension of the light irradiation using the probe detection sensor 14.

Further, there can be considered a case where the light shielding by the light-shielding member 11 is incomplete. That is, there is a possibility that a gap is formed in a part of the light-shielding member 11 due to the state of molding or the individual difference of the cover 10. In addition, the light-shielding effect can be reduced due to a reduction in flexibility resulting from the age deterioration of the light-shielding member 11 as the deformable body, or deformation or exfoliation caused by the contact with the photoacoustic probe 4. Even in such cases, by controlling the shutter 12 to suspend the light irradiation itself, it is possible to prevent even more the light leakage and enhance the safety.

Although the shutter 12 is provided inside the first illumination optical system 2, the position of the shutter 12 is not limited thereto, and the shutter 12 may be provided inside the light source 1 or at any position between the light source 1 and the second illumination optical system 5 (not shown in FIG. 3).
In addition, the controller 13 can use various methods in order to disable or enable the light irradiation. For example, instead of using the shutter 12, the light irradiation may be controlled by the control by the Q-switch of the light source 1 or by the control of the passage of electric current to the light source 1.

### <Embodiment 2>

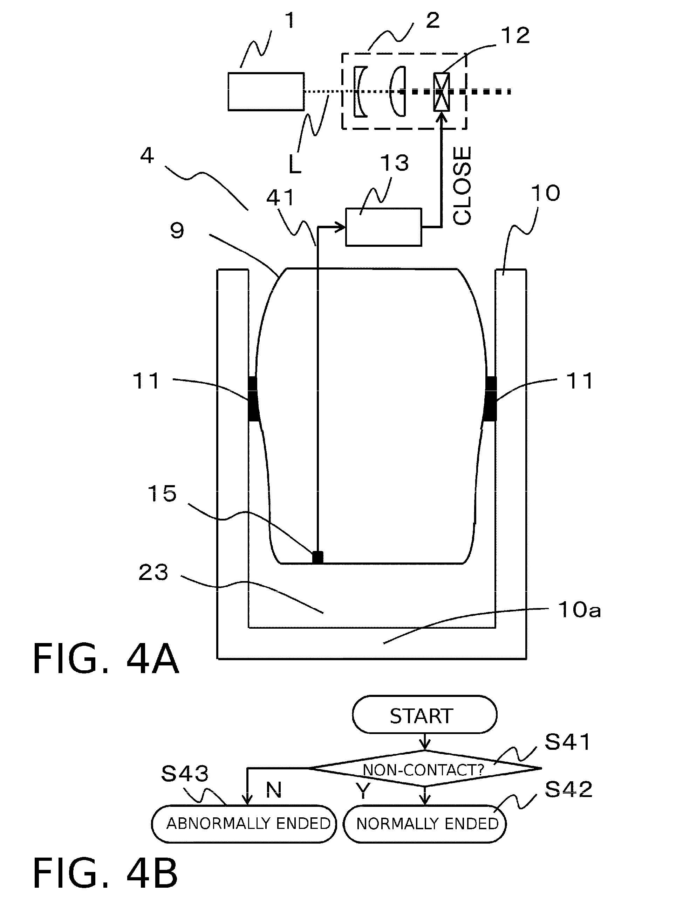

In the present embodiment, an example of the control of the light irradiation of the photoacoustic probe 4 will be described with reference to FIG. 4.

The light source 1 and the illumination light L in FIG. 4A are the same as those in FIG. 1. In addition, the first illumination optical system 2 has the shutter 12, and blocks the light by closing the shutter 12 in response to the shutter close instruction (CLOSE) from the controller 13 as described later. The photoacoustic probe 4 in FIG. 4A is considered to be substantially the same as that of FIG. 2 though the details thereof are omitted, and a contact detection sensor 15 described later is provided at the tip of the photoacoustic probe 4.

The contact detection sensor 15 provided in the photoacoustic probe 4 detects whether or not the tip of the photoacoustic probe 4 is in contact with the object 20. The contact detection sensor 15 sends contact state information 41 indicative of the contact state between the photoacoustic probe 4 and the object 20. The contact state information 41 includes non-contact information indicative of a state in which the photoacoustic probe 4 and the object 20 are not in contact with each other and contact information indicative of a state in which the photoacoustic probe 4 and the object 20 are in contact with each other.

The controller 13 controls the opening/closing operations of the shutter 12 according to the contact state information 41. That is, in a case where the non-contact information is received as the contact state information 41, the controller 13 closes the shutter 12 to prevent the irradiation from the tip of the photoacoustic probe 4. Conversely, in a case where the contact information is received as the contact state information 41, the controller 13 opens the shutter 12 to allow the illumination light emitted from the light source 1 to be emitted from the tip of the photoacoustic probe 4.

At this point, when the distance between the contact detection sensor 15 and a bottom surface 10a of the cover 10 is short, the possibility is increased that the contact detection sensor 15 erroneously detects that the photoacoustic probe 4 and the object 20 are in contact with each other. Consequently, it is necessary to form a sufficient gap 23 between the contact detection sensor 15 and the bottom surface 10a of the cover 10.

In a case where the mechanical sensor such as the limit switch or a strain gauge is used as the contact detection sensor 15, the contact detection sensor 15 may not be in contact with the bottom surface 10a of the cover 10 appropriately so that it is only necessary to provide the gap 23 such that they don't interfere with each other.
In addition, in a case where a capacitive sensor is used as the contact detection sensor 15, the gap 23 that sufficiently reduces its capacity is provided.

Further, in a case where the optical sensor is used as the contact detection sensor 15, the gap 23 is provided such that light that returns after being reflected by its light reception section becomes sufficiently reduced. Alternatively, the light of the optical sensor may be prevented from returning to the light reception section by tilting the bottom surface 10a of the cover 10. Similarly, the light of the optical sensor may be prevented from returning to the light reception section by frosting the bottom surface 10a of the cover 10 using deep colors by painting or plating, or by sandblasting the surface.

According to the configuration described above, since the contact detection sensor 15 sends the non-contact information to the controller 13 when the tip of the photoacoustic probe 4 is covered with the cover 10, the controller 13 closes the shutter 12. As a result, since it is possible to prevent the irradiation from the emission end when the tip of the photoacoustic probe 4 including the emission end of the illumination light is covered with the cover 10, the safety is improved.
On the other hand, in a case where the photoacoustic probe 4 is pushed against the object to perform the photoacoustic measurement, the shutter opening control is performed based on the contact information so that it is possible to perform the light irradiation with no problem.

Although the shutter 12 is provided inside the first illumination optical system 2, the position of the shutter 12 is not limited thereto, and the shutter 12 may be provided inside the light source 1 or at any position between the light source 1 and the second illumination optical system 5 (not shown in FIG. 4). Alternatively, instead of the shutter 12, the light may be controlled by the control by the Q-switch of the light source 1 or the control of the passage of electric current to the light source 1.

In addition, the contact detection sensor 15 can break down due to the short circuit of wiring or a substrate, and hence it is preferable to perform a fault diagnosis periodically, when the apparatus is started, or before the object is measured.

The flowchart of FIG. 4B shows the process of the fault diagnosis. The controller 13 starts the fault diagnosis of the contact detection sensor 15 in the state in which the tip of the photoacoustic probe 4 is covered with the cover 10.
In Step S41, the controller 13 confirms whether or not the contact detection sensor 15 outputs the non-contact information. When the contact detection sensor 15 outputs the non-contact information (S41 = Y), the process advances to Step S42, and the fault diagnosis process is normally ended. On the other hand, when the contact detection sensor 15 outputs the contact information (S41 = N), the process advances to Step S43, the fault diagnosis process is abnormally ended, and an alarm about the fault of the contact detection sensor 15 is issued.

Thus, it is possible to perform the fault diagnosis of the contact detection sensor 15 in the state in which the tip of the photoacoustic probe 4 is covered with the cover 10 so that it becomes possible to not only improve the safety but also smoothly perform the inspection before use.

Note that, in the present embodiment as well, similarly to Embodiment 1, since the light is not emitted when the photoacoustic probe 4 is accommodated, it is possible to obtain the effect of improving the safety even in the case where the light-shielding member 11 is not present. Further, it is possible to enhance the effect by the combination of the installation of the light-shielding member 11 and the irradiation suspension control by the controller.

### <Embodiment 3>

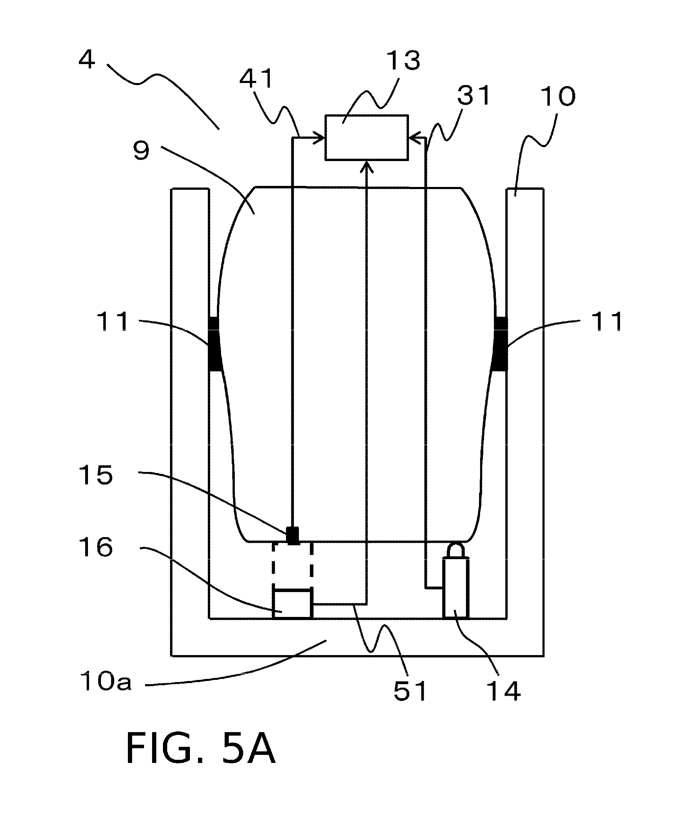

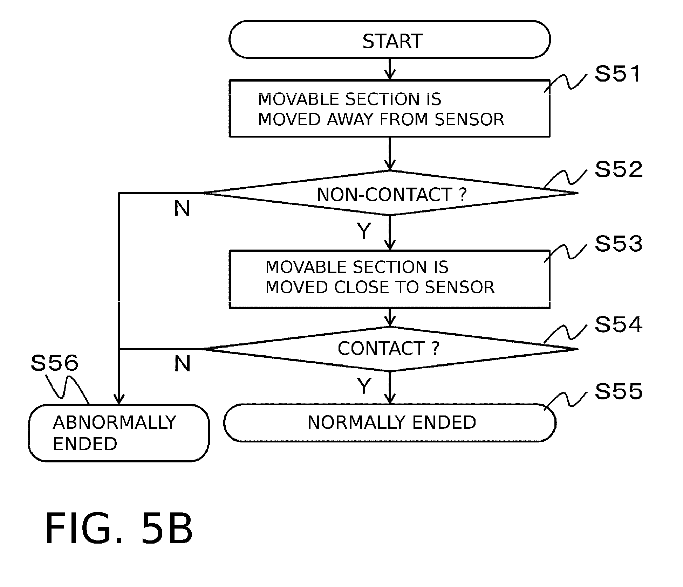

In the present embodiment, the control of the light irradiation of the photoacoustic probe 4, in particular, an example of a determination method of the fault of the probe will be described with reference to FIGS. 5A and 5B.

In Embodiment 2, the contact detection sensor 15 determines the contact state between the photoacoustic probe 4 and the cover 10 and outputs the contact state information 41, and the controller 13 performs the fault diagnosis based on whether or not the non-contact information is outputted as the contact state information 41. In the present embodiment, the fault diagnosis is performed further by using the contact information of the contact state information 41.

The photoacoustic probe 4 in FIG. 5A is considered to be substantially the same as that of FIG. 2 though the details thereof are omitted, the probe detection sensor 14 is provided inside the cover 10, and the contact detection sensor 15 is provided at the tip of the photoacoustic probe 4.

In addition, in the present embodiment, a movable section 16 is provided on the bottom surface 10a of the cover 10. The movable section 16 is provided at a position that opposes the contact detection sensor 15 when the photoacoustic probe 4 is inserted into the cover 10. The movable range of the movable section 16 lies between the position close to the contact detection sensor 15 that causes the contact detection sensor 15 to output the contact information and the position away from the contact detection sensor 15 that causes the contact detection sensor 15 to output the non-contact information.

The fault diagnosis in the present embodiment is performed according to the flowchart of FIG. 5B, and the fault diagnosis of the contact detection sensor 15 is performed by the controller 13 in the state in which the tip of the photoacoustic probe 4 is covered with the cover 10.

When the process is started, first, in Step S51, the movable section 16 is moved to the position that causes the contact detection sensor 15 to output the non-contact information as the contact state information. The position of the movable section 16 is transmitted to the controller 13 as position information 51.
Next, in Step S52, the controller 13 confirms whether or not the contact detection sensor 15 outputs the non-contact information. In a case where the non-contact information is not outputted (S52 = N), the process advances to Step S56, the fault diagnosis is abnormally ended, and the alarm about the fault of the contact detection sensor 15 is outputted.
On the other hand, when S52 = Y is satisfied, the process advances to Step S53, and the movable section 16 is moved close to the contact detection sensor 15 to the position that causes the contact detection sensor 15 to output the contact information as the contact state information.

Next, in Step S54, the controller 13 confirms whether or not the contact detection sensor 15 outputs the contact information. In a case where the contact information is not outputted (S54 = N), the process advances to Step S56, the fault diagnosis is abnormally ended, and the alarm about the fault of the contact detection sensor 15 is outputted.
On the other hand, when S54 = Y is satisfied, the process advances to Step S55, and the process is normally ended.

Thus, according to the present embodiment, since it is possible to perform the fault diagnosis by using both of the contact information and the non-contact information of the contact state information 41 in the state in which the tip of the photoacoustic probe 4 is covered with the cover 10, it is possible to enhance the accuracy of the diagnosis and further improve the safety.

In the fault diagnosis of the contact detection sensor 15 according to the above-described flowchart, since the contact detection sensor 15 outputs the contact information in the state of each of S53 and S54, the state in which the irradiation of the illumination light is allowed is established.

To cope with this, in the present embodiment, as shown in FIG. 5A, the probe detection sensor 14 is provided inside the cover 10. In a case where information indicating that the tip of the photoacoustic probe 4 is covered with the cover 10 is outputted as the probe cover information 31, the controller 13 closes the shutter 12 (not shown in FIG. 5A).

With this arrangement, even when the contact information is temporarily outputted during the fault diagnosis, when the tip of the photoacoustic probe 4 is covered with the cover 10, the irradiation from the emission end is prevented. As a result, it is possible to obtain the effects of improving the safety and smoothly performing the inspection before use.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.
There is used an object information acquiring apparatus that has a light source; a photoacoustic probe including an emission end that emits light from the light source to an object and a reception section that receives an acoustic wave generated from the object to which the light is emitted; a cover covering at least the emission end of the photoacoustic probe; a light-shielding member provided inside the cover and filling a gap formed between the photoacoustic probe and the cover when the photoacoustic probe is inserted into the cover; and a processor acquiring information on the inside of the object based on the acoustic wave.

## Claims

1. An object information acquiring apparatus comprising:
a light source;
a photoacoustic probe including an emission end that emits light from the light source to an object and a reception section that receives an acoustic wave generated from the object to which the light is emitted;
a cover configured to cover at least the emission end of the photoacoustic probe;
a light-shielding member provided inside the cover and filling a gap formed between the photoacoustic probe and the cover when the photoacoustic probe is inserted into the cover; and
a processor configured to acquire information on an inside of the object based on the acoustic wave.

2. The object information acquiring apparatus according to claim 1, wherein the light-shielding member is formed of a deformable body that is deformed into a shape surrounding the photoacoustic probe when the photoacoustic probe is inserted into the cover.

3. A cover for a photoacoustic probe including an emission end that emits light from a light source to an object and a reception section that receives an acoustic wave generated from the object to which the light is emitted,
the cover being formed to cover at least the emission end of the photoacoustic probe, and
a light-shielding member that fills a gap formed between the photoacoustic probe and the cover when the photoacoustic probe is inserted into the cover being provided inside the cover.

4. An object information acquiring apparatus comprising:
a light source;
a photoacoustic probe including an emission end that emits light from the light source to an object and a reception section that receives an acoustic wave generated from the object to which the light is emitted;
a cover configured to cover at least the emission end of the photoacoustic probe;
a probe detection sensor provided inside the cover and detecting whether or not the emission end is covered with the cover;
a controller configured to control light emission from the light source according to an output from the probe detection sensor; and
a processor configured to acquire information on an inside of the object based on the acoustic wave.

5. The object information acquiring apparatus according to claim 4, wherein the controller implements control to disable the light emission in a case where the emission end is covered with the cover.

6. The object information acquiring apparatus according to claim 4 or 5, wherein the controller controls the light emission by using any of a control of a shutter, a control of a Q-switch, and a control of passage of electric current to the light source.

7. The object information acquiring apparatus according to any one of claims 4 to 6, wherein the probe detection sensor is any of a mechanical sensor, an optical sensor, and an electrostatic sensor.

8. An object information acquiring apparatus comprising:
a light source;
a photoacoustic probe including an emission end that emits light from the light source to an object and a reception section that receives an acoustic wave generated from the object to which the light is emitted;
a cover configured to cover at least the emission end of the photoacoustic probe;
a contact detection sensor provided in the photoacoustic probe and detecting a contact between the photoacoustic probe and the object;
a controller configured to control light emission from the light source according to an output of the contact detection sensor; and
a processor configured to acquire information on an inside of the object based on the acoustic wave.

9. The object information acquiring apparatus according to claim 8, wherein the controller implements control to disable the light emission in a case where non-contact information indicating that the photoacoustic probe and the object are not in contact with each other is outputted from the contact detection sensor.

10. The object information acquiring apparatus according to claim 8 or 9, wherein the controller implements control to enable the light emission in a case where contact information indicating that the photoacoustic probe and the object are in contact with each other is outputted from the contact detection sensor.

11. The object information acquiring apparatus according to any one of claims 8 to 10, wherein a gap is formed between the contact detection sensor and an inner wall of the cover that the contact detection sensor opposes when the photoacoustic probe is inserted into the cover.

12. The object information acquiring apparatus according to any one of claims 8 to 11, wherein the controller performs a fault diagnosis of the contact detection sensor based on the output of the contact detection sensor.

13. The object information acquiring apparatus according to any one of claims 8 to 12, wherein the controller determines that the contact detection sensor is not faulty in a case where non-contact information indicating that the photoacoustic probe and the object are not in contact with each other is outputted from the contact detection sensor in a state in which the photoacoustic probe is inserted into the cover.

14. The object information acquiring apparatus according to claim 13, wherein
a movable section movable between a position close to the contact detection sensor and a position away from the contact detection sensor is provided at a position on an inner wall of the cover that opposes the contact detection sensor when the photoacoustic probe is inserted into the cover, and
the controller determines that the contact detection sensor is not faulty in a case where contact information indicating that the photoacoustic probe and the object are in contact with each other is outputted from the contact detection sensor in a state in which the photoacoustic probe is inserted into the cover and in which the movable section is at the position close to the contact detection sensor.

15. The object information acquiring apparatus according to claim 13 or 14, further comprising:
a probe detection sensor provided inside the cover and detecting whether or not the emission end is covered with the cover, wherein
the controller controls the light emission from the light source according to an output from the probe detection sensor.

16. The object information acquiring apparatus according to any one of claims 8 to 15, wherein the contact detection sensor is any of a mechanical sensor, an optical sensor, and an electrostatic sensor.
